# EUROPEAN PATENT SPECIFICATION

(11) **EP 2 884 992 B1**
(45) Date of publication and mention of the grant of the patent: **31.05.2017**
(21) Application number: 13879335.1
(22) Date of filing: 22.04.2013
(51) Int. Cl.: A61K 38/18, A61K 38/22, C12N 15/09

(54) **A METHOD OF PREPARING A GROWTH FACTOR CONCENTRATE DERIVED FROM HUMAN PLATELETS**
VERFAHREN ZUR HERSTELLUNG EINES WACHSTUMSFAKTORKONZENTRAT AUS HUMANEN THROMBOZYTEN
PROCÉDÉ DE PRÉPARATION D'UN CONCENTRÉ DE FACTEURS DE CROISSANCE OBTENU À PARTIR DE PLAQUETTES HUMAINES

(30) Priority: 17.08.2012 IN 2406MU2012
(43) Date of publication of application: 24.06.2015
(73) Proprietor: Kasiak Research Pvt. Ltd., Mumbai 400 021 Maharashtra (IN)
(72) Inventor: TOTEY, Satish Mahadeorao, Thane (W) 400610 (IN); MANIYAR, Rachana Rajiv, Mumbai-400006 (IN); FONSECA, Lyle Carl, Mumbai-400049 (IN); LAGHATE, Sneha Deepak, Mumbai 400069 (IN); KOSHY, Nicole, hane-401107 (IN)
(74) Representative: Carpintero Lopez, Francisco
(86) International application number: PCT/IN2013/000266
(87) International publication number: WO 2014/027362

(56) References cited:
- EP-A1- 2 389 942
- US-A1- 2006 142 198
- US-A1- 2010 159 022
- US-A1- 2010 273 141
- US-A1- 2012 195 946
- US-A1- 2013 195 959
- RODELLA LUIGI FABRIZIO ET AL: "Growth factors, CD34 positive cells, and fibrin network analysis in concentrated growth factors fraction.", MICROSCOPY RESEARCH AND TECHNIQUE AUG 2011, vol. 74, no. 8, August 2011 (2011-08), pages 772-777, XP002749090, ISSN: 1097-0029
- Borzini P. ET AL: "Platelet-rich plasmA (PRP) and platelet derivatives fron topical therapy. What is true from the biologic view point?", ISBT Science Series, 1 January 2007 (2007-01-01), pages 271-281, XP055025170, Retrieved from the Internet: URL:http://onlinelibrary.wiley.com/store/1 0.1111/j.1751-2824.2007.00085.x/asset/j.17 51-2824.2007.00085.x.pdf?v=1&t=h191es6g&s= 90482ddd157d28e76227c469449bce64960856e8 [retrieved on 2012-04-20]

## Description

### FIELD OF THE INVENTION

This invention relates to a method of preparing a growth factor concentrate derived from human platelets. The invention also relates to the growth factor concentrate, a lyophilized preparation thereof, a composition comprising the growth factor concentrate, a method of treating dermatological, orthopedic, neurological and endocrinological conditions, and use of the growth factor concentrate.

### BACKGROUND OF THE INVENTION

Platelets, on activation, are known to release growth factors, cytokines, chemokines and adhesive proteins. These growth factors, cytokines, chemokines and adhesive proteins play an important role in reducing inflammation and increasing proliferative events that help in tissue remodeling and regeneration of tissue in case of injuries or wounds. However, *in vivo*, matrix metalloproteinases (MMPs), which usually play an important role in protein turnover during tissue formation, can also cause non-specific tissue degradation if a high concentration of MMPs is present in the wound site for a prolonged duration. This can result in 'off target' destruction of growth factors that are essential for healing. Proteases present in the wound exudate and bacterial contamination in the wound further degrade the growth factors and can make wound healing impossible leading to a chronic, un-healing wound.

In order to jump-start the healing process, the use of additional growth factors synthesised by recombinant techniques or derived from any other source has recently been adopted in medical practices. One such source was found to be the patient's own platelets. Platelet-rich plasma (PRP) therapy has been developed and is commonly being used for various clinical indications including chronic wounds. PRP is, by definition, a volume of the plasma fraction of autologous blood having a platelet concentration above baseline or an increased concentration of autologous platelets suspended in a small amount of plasma after centrifugation. Basically, the patient's blood is collected and centrifuged at varying speeds to obtain PRP. Usually two spins are used for preparation of PRP. The first centrifugation, also known as a soft spin done at about 245 g to 900 g for 10 minutes, separates the RBC fraction, the WBC fraction and the fraction of platelets in plasma. The second spin, also known as the hard spin done at about 1500 g to 3000 g for 10 minutes, separates the platelets from the PPP. The material with the highest specific gravity i.e. the platelets will settle at the bottom of the tube, this platelet pellet is re-suspended in a small volume of plasma, generally 5 times lesser than the volume of whole blood processed, thus obtaining a PRP suspension. Immediately prior to application, a platelet activator/agonist, such as bovine thrombin and/or 10% calcium chloride, is added to activate the clotting cascade, producing a platelet gel or clot. The activated platelets release growth factors into the plasma. The whole process takes approximately 30 to 60 minutes and produces a platelet concentration of three to eight times that of native plasma. (Marx RE, Carlson ER, Eichstaedt RM, Schimmele SR, Strauss JE, Georgeff KR. Platelet-rich plasma: Growth factor enhancement for bone grafts. Oral Surg. Oral Med. Oral Pathol. Oral Radiol. Endod. 1998;85:638-646; and Petrungaro PS. Using platelet-rich plasma to accelerate soft tissue maturation in esthetic periodontal surgery. Compend. Contin. Educ. Dent. 2001;22:729-732, 734, 736 passim; quiz 746).

PRP contains not only a high level of platelets, but also contains the full complement of clotting factors since it also comprises plasma. For functional PRP, the platelets need to be activated by addition of thrombin and calcium chloride so that the platelet contents are released through degranulation of the platelet alpha granules which contain the growth factors. However, usually bovine thrombin is used for this purpose which may cause immune reactions and also carries a threat of transmission of bovine viruses or diseases to humans. The use of human thrombin makes the product uneconomical due to its high cost.

Further, clinical use of PRP for nerve injury and sports medicine has produced inconsistent results in early trials. In fact there are very few controlled clinical trials that have adequately evaluated the safety and efficacy of PRP treatments and these trials have generally concluded that PRP is a 'promising' but not 'proven' treatment option for joint, tendon, ligament, and muscle injuries. The reason for such inconsistent results and negative outcome for PRP clinical studies are associated with poor quality of PRP produced by non-standardized methods. Another problem is that most PRP preparing devices do not count the platelets and produce PRP with only a fixed percentage of platelets which is directly related to the percentage present in the original blood sample. Therefore, the actual number of platelets in PRP of different patients will be different since there is significant inter-individual variability in the platelet concentration of human plasma. This variable may affect the results since, on one hand, higher concentration of growth factors is shown to have inhibitory effects, and on the other hand PRP with lower platelet numbers may not show optimum results. Thus, there is need for a product with a fixed or standardized concentration of platelets or growth factors to be commercially available so that fixed dosages of the same can be administered to patients for the treatment of various disease conditions.

One of the major drawbacks of methods that are currently being used is that PRP can only be used as an autologous therapy and cannot be used as an allogeneic therapy because of immunological factors such as ABO incompatibility wherein donor plasma or donor platelet cell membranes may be immunogenic and cause serious adverse events in patients. Further, when PRP is administered to a patient, it is the entire intact platelets that are injected and hence the existing platelet cell membranes may cause allo-immunization and adverse reactions. Also, PRP may contain hemolysed or intact RBCs that can cause inflammatory reactions. The proportion of white blood cells, growth factors, and other chemicals such as thrombin present in PRP can also affect the final PRP quality. Another drawback of the existing method for preparing PRP is that it is a single blood draw procedure for a single dose and hence multiple blood draws are needed in case multiple doses are required. Yet another drawback is that PRP has to be used within four hours of processing and cannot be stored because storage causes flocs and fibrin clots to be formed in the plasma.

Another theory behind why PRP does not work as well as expected is that the concentration of platelets in the PRP obtained from automated machines is too low to achieve the desired clinical effect, probably because 30 to 35% of platelets are lost during processing in automated machines.

Although recently, a new substance called human platelet lysate (HPL) has been developed which has similar characteristics of PRP, it has not been used for clinical applications because it has several drawbacks including variable platelet numbers and growth factor levels. Preparation of HPL is time consuming as it involves multiple freeze-thaws of PRP for activation of the platelets to release the growth factors as a low amount of growth factors are released in a single cycle of freezing and thawing. Freezing, which causes activation and subsequent lysis of the platelet cell membranes, is usually done at -20 to -80°C and can take several hours. Thawing is usually done at 4 °C or at room temperature. Multiple freeze-thaws and long-term storage cause denaturation of the growth factors and also causes a fibrin clot to be formed on storage, making HPL unsuitable for use. Majority of the growth factors get trapped in the clot formed, thus, leaving insufficient growth factors in the plasma lysate prepared for downstream applications. Recently, a technology for removal of fibrin present in the plasma has been identified, however the method for fibrin removal is complicated and time consuming and hence makes the product unaffordable. Animal blood has also been used to make HPL and this is unsuitable for human use as it can cause xenogeneic immune reactions. Also, HPL is conventionally used in cell culture media and is not used for any therapeutic purposes/clinical applications. Further, solvent or detergents are often used for making the platelet concentrates before lysis, and the use of such solvents or detergents also makes these methods unacceptable for use of HPL in injectables for treatment of humans.

### SUMMARY OF THE INVENTION

According to an embodiment of the invention there is provided a method of preparing an intra-dermally, sub-dermally, intra-articularly or topically administrable growth factor concentrate derived from human platelets comprising the following steps:
a. suspending human platelets in an isotonic medium;
b. snap-freezing the suspension;
c. thawing the frozen suspension; and
d. sterile-filtering the suspension
wherein, a fixed number of platelets is suspended in a fixed volume of the isotonic medium to obtain the required concentration of growth factors in the growth factor concentrate;
the snap-freezing is carried out at a temperature of -120°C to -200°C;
the thawing is carried out at 25°C to 37°C; and
cellular debris are separated from the thawed suspension and the resultant suspension of growth factors is diluted with the isotonic medium before sterile-filtering and optionally lyophilized with excipients after sterile-filtering,
provided that where the isotonic medium in step (a) is plasma, the volume of plasma does not exceed 5 ml.

According to yet another embodiment of the invention there is provided a dosage of an intra-dermally, sub-dermally, intra-articularly or topically administrable growth factor concentrate prepared by a method as claimed in claim 1, derived from approximately 1250 × 10⁶ human platelets per ml, the concentrate comprising approximately 900 to 2000 pg/ml of Epidermal growth factor (EGF), 30 to 300 pg/ml of Vascular Endothelial growth factor(VEGF), 20 to 100 pg/ml of Basic fibroblast growth factor (b-FGF), 40000 to 120000 pg/ml of Transforming growth factor-β (TGF- β) and 200000 to 600000 pg/ml of Platelet Derived growth factor-AB (PDGF-AB) suspended in an isotonic medium; from approximately 875 ×10⁶ human platelets per ml, the concentrate comprising approximately 800 to 1200 pg/ml of EGF, 20 to 80 pg/ml of VEGF, 15 to 30 pg/ml of b-FGF, 30000 to 40000 pg/ml of TGF- β and 100000 to 200000 pg/ml of PDGF-AB suspended in an isotonic medium; from approximately 625 ×10⁶ human platelets, the concentrate comprising approximately 500 to 1000 pg/ml of EGF, 10 to 20 pg/ml of VEGF, 10 to 25 pg/ml of b-FGF, 20000 to 30000 pg/ml of TGF- β and 60000 to 150000 pg/ml of PDGF-AB suspended in an isotonic medium. It is to be understood that both the foregoing general description and the following detailed description of the present embodiments of the invention are intended to provide an overview or framework for understanding the nature and character of the invention as it is claimed. The accompanying graphical and pictorial representations are included to substantiate the invention and are incorporated into and constitute a part of this specification.

### BRIEF DESCRIPTION OF THE DRAWINGS

FIG. 1A shows the relation between centrifugation speed and average platelet recovery after the 1^{st} centrifugation.
FIG. 1B shows the relation between centrifugation speed and average platelet recovery after the 2^{nd} centrifugation.
FIG. 2 shows a comparison of the concentrations of various growth factors obtained by the method of the invention when the isotonic medium is multiple electrolyte isotonic solution (Example 2) as against platelet free plasma (Example 1).
FIG. 3 is a comparison of the levels of growth factors obtained by the process of Example 1 and 2 vis-à-vis the process of Example 3 as determined by ELISA.
FIG 4 shows that the levels of the various growth factors in the GFC were found to be linearly related to the concentration of platelets from which the GFC was derived.
FIG. 5 shows a comparison of the concentrations of various growth factors obtained by the method of the invention (GFC), as against growth factors obtained from platelets activated with 150 units/ml of bovine thrombin in 10% calcium chloride (PRP).
FIG. 6 shows a comparison of the concentrations of various growth factors by ELISA obtained by the method of the invention where the freezing temperature is -196°C, as compared to multiple freeze-thaws at freezing temperatures of -80°C and -20°C.
FIG 7 is a photographic comparison of a study subject showing reduced nasolabial wrinkles after three months of receiving one intra-dermal injection of growth factor concentrate derived from 625 ×10⁶ platelets per ml.
FIG 8 is a photographic comparison of a study subject showing hair growth two months after completion of treatment with growth factor concentrate, the treatment consisting of three intradermal injections of growth factor concentrate derived from 875 ×10⁶ platelets per ml, each subsequent injection being given at an interval of one month from the previous injection.
FIG 9 is a graphical comparison of Patient Rated Tennis Elbow Evaluation (PRTEE) scores of a study subject suffering from tennis elbow one month after completion of treatment with growth factor concentrate, the treatment consisting of one intra-articular injection of growth factor concentrate derived from 1250 × 10⁶ platelets per ml.
FIG 10 is a photographic comparison of a study subject showing reduced periorbital hyperpigmentation three months after completion of treatment with growth factor concentrate, the treatment consisting of one intra-dermal injection of growth factor concentrate derived from 1250 × 10⁶ platelets per ml.
FIG 11 is a photographic comparison of Wistar rats showing healing of hot paraffin wax induced burn injury 20 days after treatment with growth factor concentrate compared to untreated control, the treatment consisting of daily topical application of growth factor concentrate derived from 1250 × 10⁶ platelets per ml obtained by the process of Example 1 and 2.

### DETAILED DESCRIPTION OF THE PREFERRED EMBODIMENTS

For simplicity, and illustrative purposes, the present invention is described by referring mainly to exemplary embodiments thereof. In the following description, numerous specific details are set forth in order to provide a thorough understanding of the present invention. It will be apparent, however, to one of ordinary skill in the art that the present invention may be practiced without limitation to these specific details. In other instances, well known methods have not been described in detail so as not to unnecessarily obscure the present invention.

In the context of the invention, the term "growth factor concentrate" or "GFC" as used in the specification refers to a standardized concentration of growth factors prepared according to an embodiment of the invention, the growth factors being derived from the cryo-stimulation of platelets which have been counted, the platelets being sourced from human blood. The GFC also contains cytokines, chemokines, adhesive proteins and other modulatory peptides.

Also, "multiple electrolyte isotonic solution" as used in the specification in the context of the invention comprises sodium chloride, (NaCl); sodium gluconate (C₆H₁₁NaO₇); sodium acetate trihydrate, (C₂H₃NaO₂•3H₂0); potassium chloride, (KCl); and magnesium chloride, (MgCl₂•6H₂O) and human serum albumin. Preferably, each 100 ml of multiple electrolyte isotonic solution contains 526 mg of sodium chloride, (NaCl); 502 mg of sodium gluconate (C₆H₁₁NaO₇); 368 mg of sodium acetate trihydrate, (C₂H₃NaO₂•3H₂O); 37 mg of potassium chloride, (KCl); and 30 mg of magnesium chloride, (MgCl₂•6H2O) and 1 to 5% of human serum albumin. The pH is adjusted with sodium hydroxide to 7.4 (or 6.5 to 8.0), sterile filtered and tested for endotoxin.

Further, the term "platelet free plasma" as used in the specification in the context of the invention comprises the supernatant collected from plasma that has been centrifuged at about 17610 g and then sterile filtered. The term "platelet poor plasma" or "PPP" as used in the specification in the context of the invention comprises supernatant collected from whole blood that has been centrifuged at about 2720 g.

Freshly collected platelets or fresh blood can be collected from donors or even blood banks for large scale manufacturing of GFC. The blood is preferably transported to the central processing laboratory at 20-24°C in a transportation box. Blood can alternatively be collected from donors requiring treatment with the GFC. Blood may also be collected from other mammalian species such as horse, dog, cat, buffalo, cow, sheep, goat, rodents etc. from either jugular vein or the cephalic vein or femoral vein. One part of the blood sample collected is preferably routinely processed for complete blood count (CBC), and rapid infectious disease marker testing for Human Immunodeficiency Virus (HIV-1,2), Hepatitis B virus (HBV), Hepatitis C Virus (HCV), Venereal disease research laboratory (VDRL) tests. The remaining part of the blood collected is preferably sent to a class B environment clean-room for further processing to yield the GFC. The clean room temperature is preferably maintained at 22°C with a relative humidity of 55 %.

Stability of the platelets in the whole blood (in terms of growth factor levels) was checked for different temperatures and time points and it was found that platelets in whole blood are stable between 15 to 30°C until 24 hours for the purpose of GFC preparation i.e. the growth factor levels measured using ELISA after recovering platelets at different temperatures and time points remained stable upto 24 hours.

According to an embodiment of the invention, the blood sample to be used for obtaining the GFC is centrifuged at 109 g to 680 g, preferably at about 382 g for 15 minutes at 22°C for isolation of platelets. After centrifugation, three layers are observed: a top layer of yellow coloured platelet rich plasma (PRP), a middle layer of white blood corpuscles (WBC) and the bottom layer of red blood corpuscles (RBC). The top layer is aspirated carefully to maximize platelet yield, while ensuring that no WBCs are picked up, and placed in another sterile centrifuge tube. The platelet rich plasma collected is then centrifuged at 680 to 3442 g, preferably at about 2720 g for 10 minutes. This separates the PRP into a platelet pellet and a platelet poor plasma (PPP) supernatant. The entire PPP is collected in a sterile centrifuge tube and stored at room temperature for later use. Platelets of any desired concentrations could be prepared in this manner which is not possible in other known devices for obtaining PRP. For autologous use, the platelet pellet is re-suspended in the appropriate amount of isotonic medium. Preferably, the isotonic medium is platelet free plasma, platelet poor plasma, multiple electrolyte isotonic solution or combinations thereof. For allogeneic purposes, the concentrated platelet pellet can be re-suspended in 1 to 10 ml of multiple electrolyte isotonic solution or platelet free plasma which is tested for ABO compatibility before use. The platelets are then counted and further isotonic medium can be added so that the platelet numbers are adjusted to the count of approximately 250 to 5000 × 10⁶ platelets per ml; preferably approximately 1250 x 10⁶ platelets per ml, approximately 875 x 10⁶ platelets per ml or approximately 625 ×10⁶ platelets per ml. This platelet suspension is then subjected to a physiological activation by freezing at -196°C. The centrifuge tube containing the concentrated platelet suspension is placed in liquid nitrogen for 120 seconds and then subjected to a rapid thawing. Rapid thawing is done at 37°C for 120 seconds. Just one freeze-thaw cycle is sufficient to physiologically activate the platelets and causes lysis of the platelet membranes. This freeze-thaw cryo-stimulates the platelets to release growth factors. The suspension can then be mixed with 4 to 14 ml of PPP removed at the earlier step or platelet free plasma or multiple electrolyte isotonic solution and subjected to high speed centrifugation at a speed of 17610 g for 30 minutes. The last centrifugation is critical to remove all the plasma membranes or membrane antigens of platelets or debris so as to obtain an acellular solution. After high speed centrifugation supernatant is aspirated and transferred to another sterile tube. The GFC is amenable to lyophilisation after mixing the GFC with 2 to 10% mannitol, sucrose and/or glycine added to the GFC as bulking agent/lyoprotectant for lyophilisation. The lyophilised product is sealed with a flip off cap for clinical application and is stable for more than one year at 4°C. Lyophilised GFC can be reconstituted with 5-15 ml of multiple electrolyte isotonic solution. Sterile water for injection can also be used as a diluent for reconstitution, with 1% human serum albumin. Blood type matched plasma can also be used as a diluent.

In an embodiment of the invention, the platelet pellet is suspended in 1ml of multiple electrolyte isotonic solution and after the freeze-thaw, the thawed solution is then mixed with 9 ml of plasma which had been removed from the upper layer at the end of the second centrifugation.

In a preferred embodiment of the invention, the multiple electrolyte isotonic solution is supplemented with pharmaceutically acceptable excipients. Preferably, the pharmaceutically acceptable additives are selected from a group comprising of Acid Citrate Dextrose-solution A (ACD-A), Ethylenediaminetetraacetic acid (EDTA), and Citrate phosphate dextrose adenine (CPDA).

GFC prepared in plasma is useful for treatment of conditions such as burns or wounds requiring those proteins present in plasma which help in building a scaffolding for healing. GFC prepared in multiple electrolyte isotonic solution alone are helpful for allogeneic treatment or for treatment of those conditions which do not require plasma proteins, such as treatment of androgenetic alopecia.

GFC prepared in plasma according to the method of the invention after storage for 2 months at - 20°C and thawed at 37°C in a water bath remained a clear solution without any flocculation. GFC that has not been lyophilised can be stored at or below -20°C for a maximum of 6 months. GFC can be used for therapeutic purposes such as treatment of dermatological, orthopedic, neurological and endocrinological conditions including androgenetic alopecia, hair loss, periorbital hyperpigmentation, nasolabial wrinkles, facial wrinkles, acne, acne-scars, chronic wounds, tennis elbow, diabetic foot ulcers, fistulas, burn injuries and undesired age-related dermatological changes.

GFC comprises a combination of Growth Factors such as Epidermal Growth Factor (EGF), Vascular Endothelial Growth Factor (VEGF), Transforming Growth Factor-beta (TGF-β), basic fibroblast growth factor (bFGF), Insulin like growth factor-1 (IGF-1), Hepatocyte Growth Factor (HGF), Platelet derived growth Factor- (PDGF-AA), (PDGF-AB), (PDGF-BB); Cytokines like RANTES, Interleukin-1 beta (IL-1β), Macrophages inhibitory protein-1 alpha (MIP-1α), GRO-alpha, ENA-78, MCP-3, NCP, IGFBP-3; Basic Proteins like Platelet factor 4 (PF-4), Endostatin, PBP, Connective tissue activating peptide(CTAP), Neutrophil activating peptide (NAP); adhesive proteins such as ECGF, Plasminogen activator inhibitior-1, Laminin-8, Fibrinogen, Fibronectin, Thrombospondin and antimicrobial agents like Thrombocidin. These growth factors and other proteins have regenerative properties and help in initiating the healing process. The GFC alone or along with pharmaceutically acceptable excipients is formulated and administered for anti-ageing, dermatological disorders, hair loss, chronic wounds, orthopedic disorders, diabetic foot ulcers, fistulas, burn injuries, endocrinological disorders, neurological disorders, ophthalmological disorders, musculoskeletal disorders etc. The GFC prepared by the method of the present invention displays high propensity towards regeneration or rapid healing of wounds.

The GFC product was tested for endotoxin so as to confirm that the product was safe and free from bacterial contamination. The GFC product was also tested for infectious diseases like HIV, HCV, HBV, Syphilis etc and was found to be disease-free and safe for clinical applications.

Clinical studies on various indications such as Androgenetic alopecia, periorbital hyperpigmentation, nasolabial wrinkles, and tennis elbow were undertaken for the GFC prepared by the method of the present invention. In the clinical trials being conducted, administration of GFC led to a significant improvement in hair regeneration, decrease in periorbital hyperpigmentation, reduction in nasolabial wrinkles and speeding up recovery from tennis elbow In pre-clinical studies on Wistar rats, administration of GFC led to speedy healing of burn wounds compared to untreated rats.

It is within the scope of the invention to use other human cell types i.e. other than platelets as well, to yield the GFC according to the method of the invention It is also within the scope of the invention to use other mammalian cells or placenta to yield the GFC according to the method of the invention.

Preferably, the snap-freezing is done in liquid nitrogen or in liquid helium. The thawing can be done in a sterile water bath at 37°C. The isotonic medium can be multiple electrolyte isotonic solution, plasma, platelet free plasma, platelet poor plasma or a combination thereof. The multiple electrolyte isotonic solution can be supplemented with pharmaceutically acceptable excipients. Preferably, the excipients include mannitol, sucrose, glycine or combinations thereof.

Preferably, the cellular debris are removed from the thawed suspension by centrifuging the thawed suspension at 11270 g to 17610 g for 25 to 35 minutes and isolating the supernatant.

The platelets of step (a) of the method can be obtained by plateletpheresis, or by centrifugation of whole blood. The platelets of step (a) are preferably obtained by:
a. centrifuging at least 10 ml of anticoagulated human blood at 109 g to 680 g for 5 to 20 minutes;
b. isolating the top-most layer containing platelets and centrifuging the same at 680 g to 3442 g for 5 to 15 minutes; and
c. isolating the plasma-free pellet of platelets obtained at the end of step (b). Still preferably, the centrifugation in step (a) is carried out at 382 g for 15 minutes and the centrifugation in step (b) is carried out at 2720 g for 10 minutes.

According to another embodiment of the invention, there is provided the growth factor concentrate prepared by a method as claimed in claim 1 for use in the treatment of human dermatological, orthopedic, neurological or endocrinological conditions. The conditions can include androgenetic alopecia, hair loss, periorbital hyperpigmentation, nasolabial wrinkles, facial wrinkles, acne, acne-scars, chronic wounds, burn injuries, tennis elbow, diabetic foot ulcers, fistulas, and undesired age-related dermatological changes.

Preferably, periorbital hyperpigmentation, chronic wounds, burn injuries, tennis elbow, diabetes foot ulcer and fistulas are treated with the growth factor concentrate derived from approximately 1250 × 10⁶ human platelets per ml. Preferably, androgenetic alopecia and hair loss are treated with the growth factor concentrate derived from approximately 875 ×10 human platelets per ml. Preferably, nasolabial wrinkles, facial wrinkles, and any age related dermatological condition are treated with the growth factor concentrate derived from approximately 625 × 10⁶ human platelets per ml.

According to another embodiment of the invention, there is provided a therapeutic composition for topical, sub-dermal, intra-articular or intra-dermal administration comprising the growth factor concentrate prepared by a method as claimed in claim 1 in combination with supplemental constituents including blood, saline, silver nanoparticles, hyaluronic acid, immuno-modulatory peptides, growth factors, hormones, antibiotics, monoclonal antibodies, recombinant receptors, carriers or combinations thereof. The composition can be in the form of a cream, gel, aqueous solution, spray-aerosol or transdermal patch.

In order that those skilled in the art will be better able to practice the present disclosure, the following examples are given by way of illustration and not by way of limitation.

### Example 1

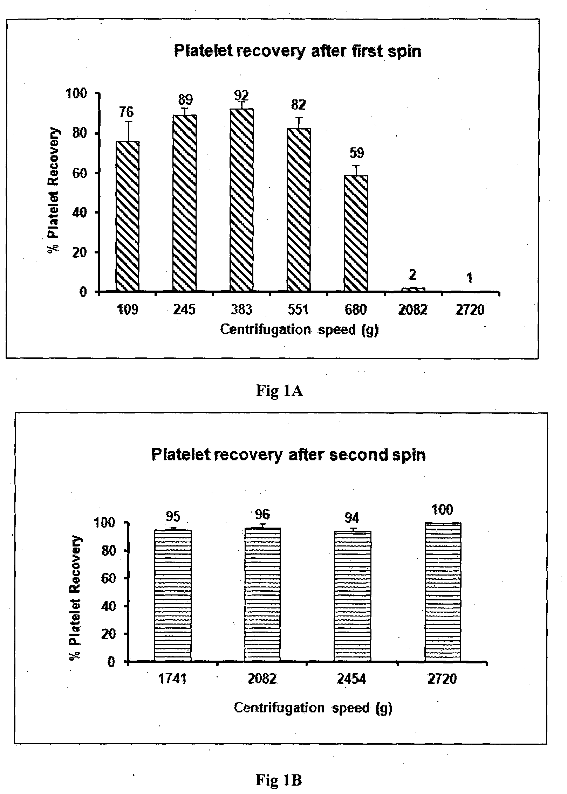

Human blood was withdrawn into vacutainers after getting informed consent from the patient. About 50-60 ml blood is collected in two types of vacutainers: 50 ml in vacutainers containing ACD-A for preparing GFC and 5-10 ml blood in EDTA tubes for infectious disease marker testing and complete blood parameter testing. Minimum 50 ml of blood was taken for 10 ml of GFC preparation. Blood was transported at 15 to 30°C preferably at 22°C within 4 hours of withdrawal. The first centrifugation was done at 382 g for 15 minutes since platelet recovery at 382 g for 15 minutes is optimum with a loss of only 8-10% platelets as shown in FIG. 1A. Platelet loss is significantly greater at lower or higher centrifugation speeds. Upon completion of first centrifugation three layers were formed. At the bottom were packed red blood cells, in the middle were leukocytes and the upper layer had plasma containing platelets. Plasma containing platelets was aspirated and transferred in another sterile tube. The second centrifugation was done at 2720 g for 10 minutes since centrifugation at 2720 g for 10 minutes results in platelet recovery of almost 99.5% as shown in FIG. 1B. The upper layer had plasma and at the bottom was a packed platelet pellet. The entire plasma (PPP) was removed and placed in a sterile tube and stored at room temperature for later use. Platelet pellet was suspended in 1 ml of PPP. Platelets were then counted and the concentration of platelets per ml of plasma was noted to be 12500×10⁶. The solution was then snap-frozen in liquid nitrogen at -196°C for 2 minutes. The frozen solution was then rapidly thawed in a water bath at 37°C for 2 minutes. In order to standardize the concentration of growth factors in the solution, the thawed solution was then mixed with 9 ml of PPP. This solution was then transferred to another sterile tube and subjected to high speed centrifugation at 17610 g for 30 minutes. The supernatant containing the growth factors was collected and sterile filtered through a 0.22micron filter.

Since the final volume of the GFC is 10ml, and the number of platelets used for preparing the GFC were 12500×10⁶, the effective concentration of platelets used for preparation of the GFC is 1250×10⁶ platelets per ml. Similarly, a final volume of the GFC of 5 ml was prepared wherein the number of platelets used for preparing the GFC were 6250 x 10⁶, so that the the effective concentration of platelets used for preparation of the GFC was still 1250 x 10⁶ platelets per ml.

### Example 2

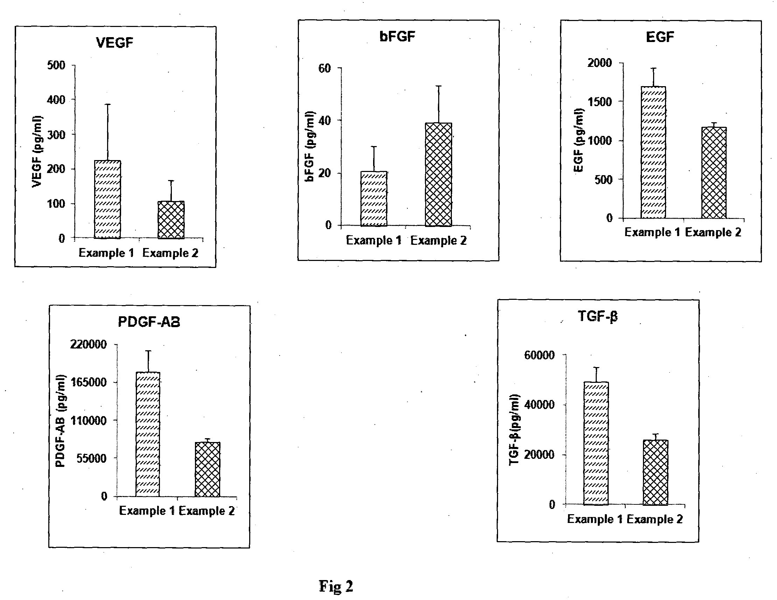

The process of Example 1 was followed except that the platelet pellet was suspended in 1ml of multiple electrolyte isotonic solution and after the freeze-thaw, the thawed solution was then mixed with 9 ml more of multiple electrolyte isotonic solution. FIG. 2 shows a comparison of the concentrations of various growth factors obtained by the process of Example 1 versus Example 2 for blood obtained from the same donor. The graphs show that levels of various growth factors like Vascular Endothelial Growth Factor (VEGF), basic Fibroblast Growth Factor (bFGF), Platelet Derived Growth Factor-AB (PDGF-AB), Epidermal Growth Factor (EGF), Transforming Growth Factor-beta (TGF-β) as determined by Enzyme linked immunosorbent assay (ELISA) were almost comparable in GFC prepared with plasma (Example 1) and GFC which is plasma free (Example 2).

### Example 3

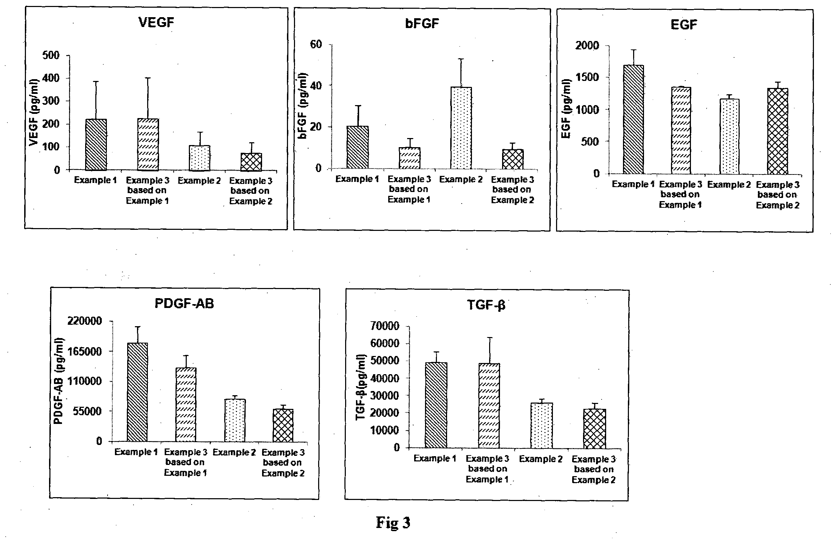

The processes of Example 1 and Example 2 were separately followed using blood obtained from the same donor. Further, the suspension containing GFC was mixed with 10% mannitol. The solution was then lyophilized. The lyophilized product was then packed in vials with flip off caps so as to make an "off the shelf" product. FIG. 3 is a comparison of the levels of growth factors obtained by the process of Example 1 and 2 vis-à-vis the process of Example 3 as determined by ELISA. It is evident that the levels of growth factors are almost the same or marginally reduced in the lyophilized GFC product as compared to the GFC prepared by Example 1 and Example 2.

### Example 4

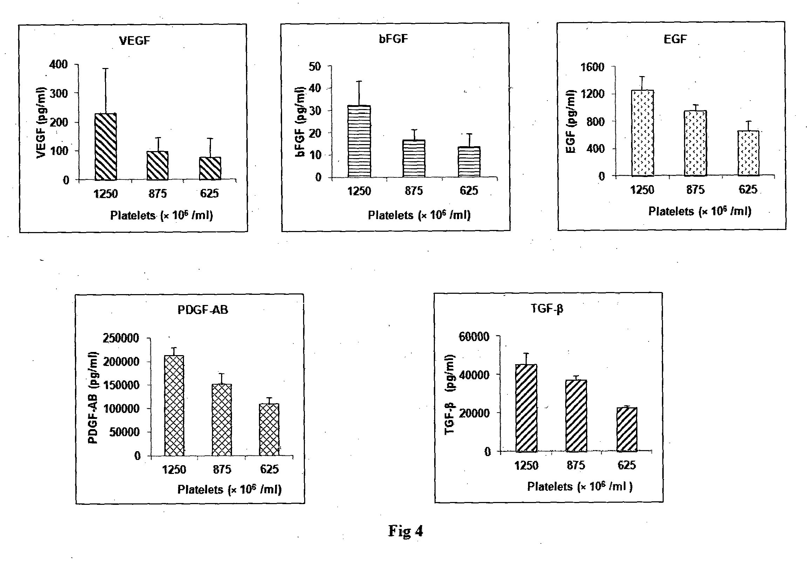

The process of Example 1 was repeated except that two more concentrations of GFC were prepared wherein the number of platelets counted and suspended in 1ml of PPP were 8750 ×10⁶ platelets per ml and 6250 ×10⁶ platelets per ml such that after mixing the supernatant containing the GFC with 9ml more of PPP, the effective concentration of platelets used for preparing the GFC were 875 ×10⁶ platelets per ml and 625 ×10⁶ platelets per ml respectively. All three concentrations of GFC i.e. GFC derived from 1250×10⁶ platelets per ml as per Example 1, and GFC derived from 875 ×10⁶ platelets per ml and 625 × 10⁶ platelets per ml as per Example 4, were made using blood from the same donor. The major growth factors present in GFC were measured by ELISA. GFC was assayed for VEGF, bFGF, PDGF-AB, EGF and TGF-β. FIG 4 shows that the levels of the various growth factors in the GFC were found to be linearly related to the concentration of platelets from which the GFC was derived.

### Example 5

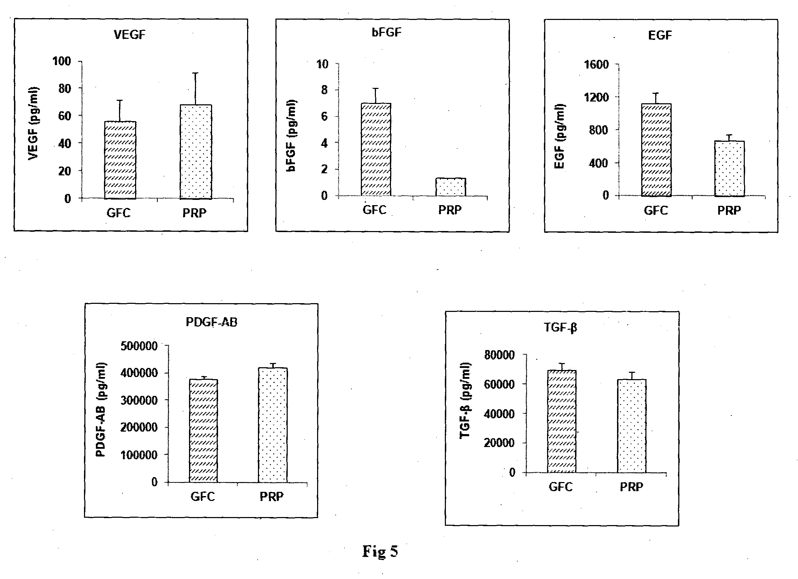

The process of Example 1 was followed up to the step of counting the platelets and making a concentration of 1250×10⁶ platelets per ml of plasma. This concentration of platelets was then activated using 150units/ml of bovine thrombin in 10% calcium chloride. After ten minutes, a clot formed and the solution was centrifuged. The supernatant was then separated and the level of growth factors therein was determined by ELISA and compared to the level of growth factors prepared by the process of Example 1 and obtained from a blood sample of the same donor. FIG. 5 shows a comparison of the concentrations of various growth factors obtained by the process of Example 1 versus the process of Example 5. FIG. 5 shows that levels of VEGF, bFGF, PDGF-AB, EGF and TGF-β were higher or comparable in GFC prepared by the process of Example 1 as compared to the levels of growth factors obtained by Example 5.

### Example 6

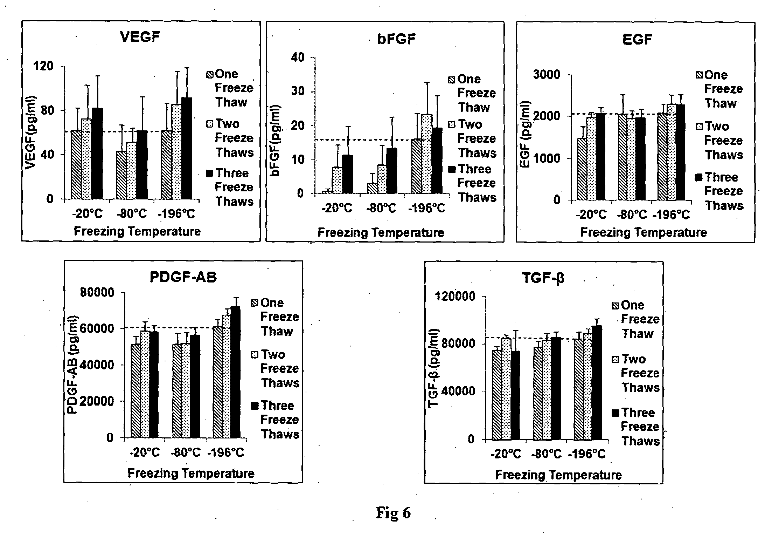

The process of Example 1 was followed up to the step of counting the platelets and making a concentration of 1250×10⁶ platelets per ml of plasma. This concentration of platelets was then activated using one freeze-thaw cycle, two freeze-thaw cycles and three freeze-thaw cycles wherein freezing was done at -20°C, -80°C and -196 °C followed by thawing at 37 °C once, twice and thrice for each temperature. After the freeze-thawing, the rest of the procedure of Example 1 was followed namely dilution, final centrifugation and sterile filtering. FIG. 6 shows a comparison of the concentrations of various growth factors by ELISA obtained in Example 6 for one freeze-thaw cycle, two freeze-thaw cycles and three freeze-thaw cycles wherein freezing was done at -20°C, -80°C and -196 °C. From the results, it is evident that one freeze-thaw cycle at -196°C is sufficient for release of growth factors as compared to two or three freeze-thaws at the freezing temperatures of the prior art ie -20 °C and -80 °C, which are also time consuming and not suitable for production purposes in which time is a very important factor. Results showed that growth factor levels increased with the number of freeze-thaw cycles at freezing temperatures of -20 °C and -80 °C and the optimum temperature of freezing was -196 °C since only one freeze-thaw cycle was required for optimum release of growth factors when freezing is done at this temperature. Also, it must be noted that repeated freeze thaws are undesirable due to denaturation of proteins when they are subjected to repeated freeze thaws.

### Example 7

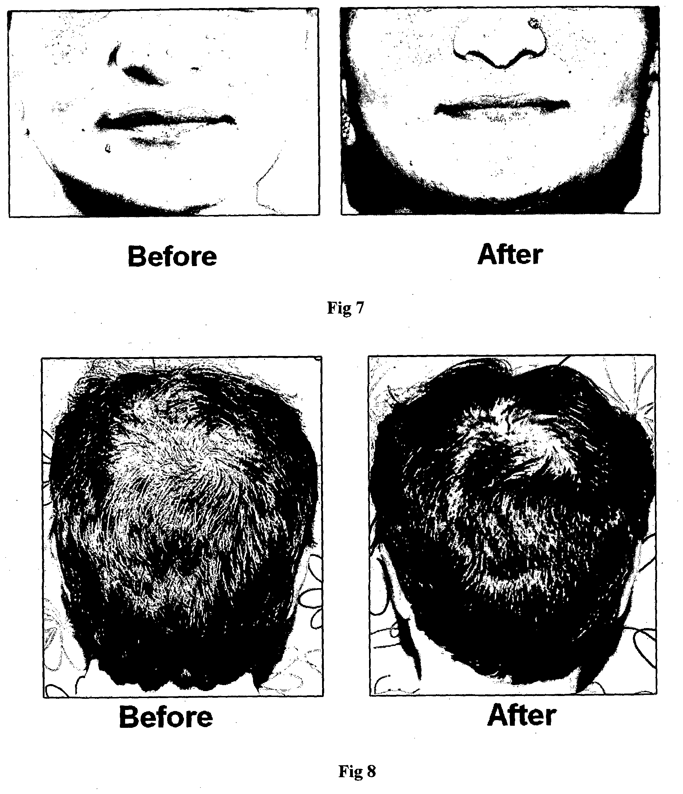

A few study subjects were treated with the GFC prepared according to Example 4 wherein the GFC was prepared from 625 ×10⁶ platelets per ml. FIG 7 is a photographic comparison of reduced nasolabial wrinkles of one study subject after three months of receiving one intradermal injection of GFC derived from 625 ×10⁶ platelets per ml. It is evident from the figure that administration of GFC produces an appreciable decrease in the prominence of nasolabial wrinkles.

### Example 8

A few study subjects were treated with the GFC prepared according to Example 4 wherein the GFC was prepared from 875 ×10⁶ platelets per ml. FIG 8 is a photographic comparison of hair growth at two months after completion of treatment with growth factor concentrate, the treatment consisting of three intra-dermal injections of growth factor concentrate derived from 875 ×10⁶ platelets per ml, each subsequent injection being given at an interval of one month from the previous injection. It is evident from the figure that administration of the growth factor concentrate produces an appreciable increase in hair growth.

### Example 9

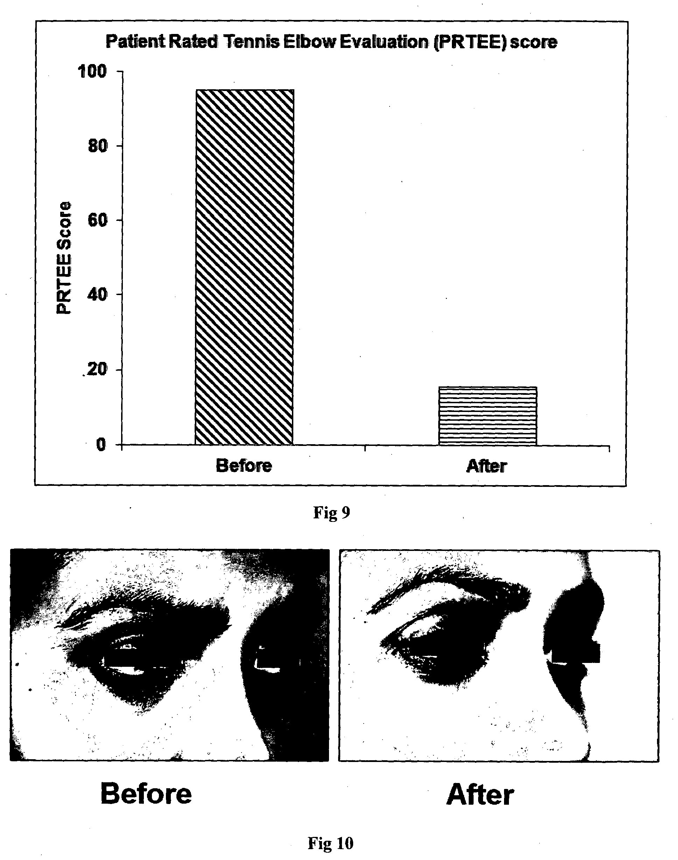

A few study subjects were treated with the GFC prepared according to Example 1 wherein the GFC was prepared from 1250 ×10⁶ platelets per ml. FIG 9 is a graphical comparison of PRTEE scores of a study subject suffering from tennis elbow one month after completion of treatment with growth factor concentrate, the treatment consisting of one intra-articular injection(s) of growth factor concentrate derived from 1250 ×10⁶ platelets per ml. It is evident from the figure that administration of the growth factor concentrate produces an appreciable decrease in the PRTEE score.

### Example 10

A few study subjects were treated with the GFC prepared according to Example 1 wherein the GFC was prepared from 1250 ×10⁶ platelets per ml. FIG 10 is a photographic comparison of reduced periorbital hyperpigmentation of one study subject three months after completion of treatment with growth factor concentrate, the treatment consisting of one intra-dermal injection of GFC derived from 1250 ×10⁶ platelets per ml. It is evident from the figure that administration of GFC produces an appreciable decrease in the periorbital hyperpigmentation around the eyes.

### Example 11

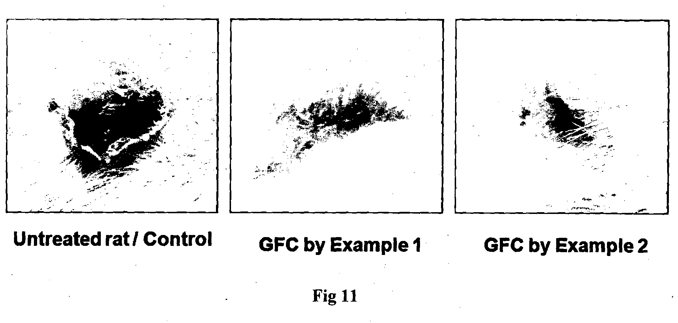

Wistar rats having hot paraffin wax induced burn injury were treated with the GFC derived from 1250 ×10⁶ platelets per ml prepared by the process of Example 1 and 2. The treatment consisted of daily topical application of the GFC. FIG 11 is a photographic comparison of the observations at the 20^{th} day after treatment with the GFC. Healing of the burn injuries was observed at the 20^{th} day in the rats treated with GFC prepared by Example 1 and 2, as opposed to the untreated rats.

As is evident from the above results, the GFC of the present invention is derived from predetermined numbers of platelets yielding proportionate amounts of growth factors which in turn serves to provide uniform clinical results. Fixed dosages of GFC can be administered to patients to treat various dermatological, orthopedic, neurological and endocrinological conditions including androgenetic alopecia, hair loss, periorbital hyperpigmentation, nasolabial wrinkles, facial wrinkles, acne, acne-scars, chronic wounds, tennis elbow, diabetic foot ulcers, fistulas, burn injuries and undesired age-related dermatological changes. Further, less than 10% platelets are lost by the method of the invention. Also, the platelets are physiologically activated without incorporating any additional materials or chemical substances, like calcium chloride or bovine thrombin, and hence it is safe. Also, the single freeze-thaw makes the process less time consuming and more suitable for large scale production and the growth factors are not denatured as in the case of multiple freeze-thaws. Moreover, some clinical indications need a large amount of growth factors but some indications need very little growth factors since the presence of growth factor receptors vary from one cell type to another and one indication to another. Therefore, the present invention provides GFCs that can have standardized concentrations which can be diluted as per the requirement to make it suitable for specific clinical indications. Another benefit of the present method is that multiple doses of GFC can be prepared from a single blood draw and hence this method is cost effective and not time consuming. Further, GFC does not show any flocculation on long-term storage for upto six months at -20°C. A small floc is sometimes seen which generally gets dissolved within 2-3 minutes at room temperature and hence it is possible to use GFC as an "off the shelf" product which can be stored for upto six months without any problem of losing its potency. Also, since the GFC is acellular and devoid of plasma membranes or other antigenic materials, it does not elicit any immune reactions or formation of allo-antibodies. GFC can optionally be made plasma free so that it can be used as a therapeutic agent without any problem of ABO incompatible plasma that may cause immune reactions. Alternatively, if the contents of plasma will serve as a beneficial scaffold in applications like wound healing, then GFC can also be prepared in ABO-matched plasma. GFC is also amenable to lyophilisation so that the GFC can be stored at room temperature or in a 4°C refrigerator without any degradation for more than one year. Further, lyophilized GFC can be made into a cream, gel, aqueous solution, spray-aerosol or transdermal patch. All in all, GFC is a natural ie non-recombinant product and the method provided by the present invention for production of GFC is economical. Further, GFC shows improved clinical outcomes due to the significantly higher level of growth factors in GFC as compared to multiple freeze-thawed HPL prepared by known methods. GFC also serves as a personalised therapy for patients requiring specific concentrations of GFC to be administered as the GFC can be prepared in any desired concentration.

## Claims

1. A method of preparing an intra-dermally, intra-articularly, sub-dermally or topically administrable growth factor concentrate derived from human platelets comprising the following steps:
a. suspending human platelets in an isotonic medium;
b. snap-freezing the suspension;
c. thawing the frozen suspension; and
d. sterile-filtering the suspension
wherein a fixed number of platelets is suspended in a fixed volume of the isotonic medium to obtain the required concentration of growth factors in the growth factor concentrate; the snap-freezing is carried out at a temperature of -120°C to -200°C; the thawing is carried out at 25°C to 37°C; and cellular debris are separated from the thawed suspension and the resultant suspension of growth factors is diluted with the isotonic medium before sterile-filtering and optionally lyophilized with excipients after sterile-filtering, provided that where the isotonic medium in step (a) is plasma, the volume of plasma does not exceed 5 ml.

2. The method as claimed in claim 1 wherein, the freezing is done in liquid nitrogen or in liquid helium.

3. The method as claimed in claim 1 wherein, the thawing is done in a sterile water bath at 37°C.

4. The method as claimed in claim 1 wherein, the isotonic medium is multiple electrolyte isotonic solution, plasma, platelet free plasma, platelet poor plasma or a combination thereof.

5. The method as claimed in claim 4 wherein, the multiple electrolyte isotonic solution is supplemented with pharmaceutically acceptable excipients.

6. The method as claimed in claim 1 wherein, the cellular debris are removed from the thawed suspension by centrifuging the thawed suspension at 1 1270 g to 17610 g for 25 to 35 minutes and isolating the supernatant.

7. The method as claimed in claim 1 wherein, the excipients include mannitol, sucrose, glycine or combinations thereof.

8. The method as claimed in claim 1 wherein, the platelets of step (a) are Obtained by plateletpheresis, or by centrifugation of whole blood.

9. The method as claimed in claim 1 wherein, the platelets of step (a) are obtained by:
a. centrifuging at least 10 ml of anticoagulated human blood at 109 g to 680 g for 5 to 20 minutes;
b. isolating the top-most layer containing platelets and centrifuging the same at 680 g to 3442 g for 5 to 15 minutes; and c. isolating the plasma-free pellet of platelets obtained at the end of step (b).

10. The method as claimed in claim 9 wherein, the centrifugation in step (a) is carried out at 382 g for 5 minutes and the centrifugation in step (b) is carried out at 2720 g for 10 minutes.

11. A dosage of an intra-dermally, intra-articularly, sub-dermally or topically administrable growth factor concentrate prepared by a method as claimed in claim 1, derived from approximately 1250 × 10⁶ human platelets per ml, the concentrate comprising approximately 900 to 2000 pg/ml of Epidermal growth factor (EGF), 30 to 300 pg/ml of Vascular Endothelial growth factor(VEGF), 20 to 100 pg/ml of Basic fibroblast growth factor (b-FGF), 40000 to 120000 pg/ml of Transforming growth factor-β (TGF- P) and 200000 to 600000 pg/ml of Platelet Derived growth factor- AB (PDGF-AB) suspended in an isotonic medium; from approximately 875 × 10⁶ human platelets per ml, the concentrate comprising approximately 800 to 1200 pg/ml of EGF, 20 to 80 pg/ml of VEGF, 15 to 30 pg/ml of b-FGF, 30000 to 40000 pg/ml of TGF- β and 100000 to 200000 pg/ml of PDGF-AB suspended in an isotonic medium; or from approximately 625 × 10⁶ human platelets, the concentrate comprising approximately 500 to 1000 pg/ml of EGF, 10 to 20 pg/ml of VEGF, 10 to 25 pg/ml of b-FGF, 20000 to 30000 pg/ml of TGF- β and 60000 to 150000 pg/ml of PDGF-AB suspended in an isotonic medium.

12. A therapeutic composition comprising the topically, intra-articularly, sub-dermally or intra-dermally administrable growth factor concentrate prepared by the method claimed in claim 1 for use in the treatment of dermatological, orthopedic, neurological and endocrinological conditions , wherein the conditions such as include androgenetic alopecia, hair loss, periorbital hyperpigmentation, nasolabial wrinkles, facial wrinkles, acne, acne- scars, chronic wounds, burn injuries, tennis elbow, diabetic foot ulcers, fistulas, and undesired age-related dermatological changes.

13. A therapeutic composition comprising the topically, intra-articularly, sub-dermally or intra-dermally administrable growth factor concentrate prepared by the method as claimed in claim 1 wherein the growth factor concentrate is in combination with supplemental constituents including blood, saline, silver nanoparticles, hyaluronic acid, immuno-modulatory peptides, growth factors, hormones, antibiotics, monoclonal antibodies, recombinant receptors, carriers or combinations thereof.

## Patentansprüche

1. Verfahren zur Herstellung eines intradermal, intraartikulär, subdermal oder topisch verabreichbaren Wachstumsfaktorkonzentrats, das aus humanen Thrombozyten stammt, umfassend die folgenden Schritte:
a. Suspendieren humaner Thrombozyten in einem isotonischen Medium;
b. Schockgefrieren der Suspension;
c. Auftauen der gefrorenen Suspension und
d. Sterilfiltration der Suspension,
wobei eine festgelegte Anzahl Thrombozyten in einem festgelegten Volumen des isotonischen Mediums suspendiert wird, so dass die erforderliche Konzentration an Wachstumsfaktoren in dem Wachstumsfaktorkonzentrat erhalten wird; das Schockgefrieren bei einer Temperatur von -120 °C bis -200 °C durchgeführt wird; das Auftauen bei 25 °C bis 37 °C durchgeführt wird; und die Zellbruchstücke von der aufgetauten Suspension getrennt werden und die resultierende Suspension aus Wachstumsfaktoren mit dem isotonischen Medium vor der Sterilfiltration verdünnt und gegebenenfalls mit Hilfsstoffen nach der Sterilfiltration lyophilisiert wird, mit der Maßgabe, dass, wenn das isotonische Medium in Schritt (a) Plasma ist, das Plasmavolumen 5 ml nicht übersteigt.

2. Verfahren nach Anspruch 1, wobei das Gefrieren in flüssigem Stickstoff oder in flüssigem Helium erfolgt.

3. Verfahren nach Anspruch 1, wobei das Auftauen in einem sterilen Wasserbad bei 37 °C erfolgt.

4. Verfahren nach Anspruch 1, wobei das isotonische Medium eine isotonische Lösung aus verschiedenen Elektrolyten, Plasma, Thrombozyten-freies Plasma, Plasma mit wenigen Thrombozyten oder eine Kombination davon ist.

5. Verfahren nach Anspruch 4, wobei die isotonische Lösung aus verschiedenen Elektrolyten mit pharmazeutisch akzeptablen Hilfsstoffen ergänzt wird.

6. Verfahren nach Anspruch 1, wobei die Zellbruchstücke aus der aufgetauten Suspension durch Zentrifugieren der aufgetauten Suspension bei 11270 g bis 17610 g für 25 bis 35 Minuten und Isolieren des Überstandes entfernt werden.

7. Verfahren nach Anspruch 1, wobei die Hilfsstoffe Mannitol, Saccharose, Glycin oder Kombinationen davon umfassen.

8. Verfahren nach Anspruch 1, wobei die Thrombozyten aus Schritt (a) durch Thrombozytopherese oder durch Zentrifugation von Vollblut erhalten werden.

9. Verfahren nach Anspruch 1, wobei die Thrombozyten aus Schritt (a) erhalten werden durch:
a. Zentrifugieren von zumindest 10 ml von ungerinnbarem humanen Blut bei 109 g bis 680 g für 5 bis 20 Minuten;
b. Isolieren der obersten Schicht, die die Thrombozyten enthält, und Zentrifugieren derselben bei 680 g bis 3442 g für 5 bis 15 Minuten; und
c. Isolieren des Plasma-freien Thrombozytenpellets, das am Ende von Schritt (b) erhalten wurde.

10. Verfahren nach Anspruch 9, wobei das Zentrifugieren in Schritt (a) bei 382 g für 5 Minuten durchgeführt wird und das Zentrifugieren in Schritt (b) bei 2720 g für 10 Minuten durchgeführt wird.

11. Dosierung eines intradermal, intraartikulär, subdermal oder topisch verabreichbaren Wachstumsfaktorkonzentrats, das mit einem Verfahren nach Anspruch 1 hergestellt wurde und aus ungefähr 1250 x 10⁶ humanen Thrombozyten pro ml, wobei das Konzentrat ungefähr 900 bis 2000 pg/ml epidermalen Wachstumsfaktor (EGF), 30 bis 300 pg/ml vaskulären Endothelwachstumsfaktor (VEGF), 20 bis 100 pg/ml basischen Fibroblasten-Wachstumsfaktor (b-FGF), 40.000 bis 120.000 pg/ml transformierten Wachstumsfaktor-β (TGF-β) und 200.000 bis 600.000 pg/ml aus Thrombozyten gewonnenen Wachstumsfaktor-AB (PDGF-AB), suspendiert in einem isotonischen Medium, umfasst; aus ungefähr 875 x 10⁶ humanen Thrombozyten pro ml, wobei das Konzentrat ungefähr 800 bis 1.200 pg/ml EGF, 20 bis 80 pg/ml VEGF, 15 bis 30 pg/ml b-FGF, 30.000 bis 40.000 pg/ml TGF-β und 100.000 bis 200.000 pg/ml PDGF-AB, suspendiert in einem isotonischen Medium, umfasst; oder aus ungefähr 625 x 10⁶ humanen Thrombozyten, wobei das Konzentrat ungefähr 500 bis 1.000 pg/ml EGF, 10 bis 20 pg/ml VEGF, 10 bis 25 pg/ml b-FGF, 20.000 bis 30.000 pg/ml TGF-β und 60.000 bis 150.000 pg/ml PDGF-AB, suspendiert in einem isotonischen Medium, umfasst, stammt.

12. Therapeutische Zusammensetzung, umfassend das topisch, intraartikulär, subdermal oder intradermal verabreichbare Wachstumsfaktorkonzentrat, das mit dem Verfahren nach Anspruch 1 hergestellt wurde, zur Verwendung bei der Behandlung dermatologischer, orthopädischer, neurologischer und endokrinologischer Zustände, wobei die Zustände androgenetische Alopezie, Haarausfall, periorbitale Hyperpigmentierung, nasolabiale Falten, Gesichtsfalten, Akne, Aknenarben, chronische Wunden, Verbrennungsverletzungen, Tennisarm, diabetische Fußgeschwüre, Fisteln und unerwünschte altersbedingte dermatologische Veränderungen umfassen.

13. Therapeutische Zusammensetzung, umfassend das topisch, intraartikulär, subdermal oder intradermal verabreichbare Wachstumsfaktorkonzentrat, das mit dem Verfahren nach Anspruch 1 hergestellt wurde, wobei das Wachstumsfaktorkonzentrat in Kombination mit ergänzenden Bestandteilen, umfassend Blut, physiologische Kochsalzlösung, Silbernanopartikel, Hyaluronsäure, immunomodulatorische Peptide, Wachstumsfaktoren, Hormone, Antibiotika, monoklonale Antikörper, rekombinante Rezeptoren, Träger oder Kombinationen davon, vorliegt.

## Revendications

1. Procédé de préparation d'un concentré de facteurs de croissance dérivé de plaquettes humaines administrable par voie intra-dermique, intra-articulaire, sous-dermique ou topique comprenant les étapes suivantes consistant à :
a. suspendre des plaquettes humaines dans un milieu isotonique ;
b. congeler rapidement la suspension ;
c. décongeler la suspension congelée ; et
d. filtrer de manière stérile la suspension ;
dans lequel un nombre fixe de plaquettes est suspendu dans un volume fixe du milieu isotonique pour obtenir la concentration nécessaire de facteurs de croissance dans le concentré de facteurs de croissance ; la congélation rapide est réalisée à une température de -120 °C à -200 °C ; la décongélation est réalisée à une température de 25 °C à 37 °C ; et les débris cellulaires sont séparés de la suspension décongelée et la suspension résultante des facteurs de croissance est diluée avec le milieu isotonique avant la filtration de manière stérile et facultativement lyophilisée avec des excipients après la filtration de manière stérile, à condition que lorsque le milieu isotonique de l'étape (a) est du plasma, le volume de plasma n'excède pas 5 ml.

2. Procédé selon la revendication 1, dans lequel la congélation est réalisée dans de l'azote liquide ou dans de l'hélium liquide.

3. Procédé selon la revendication 1, dans lequel la décongélation est réalisée dans un bain d'eau stérile à 37 °C.

4. Procédé selon la revendication 1, dans lequel le milieu isotonique est une solution isotonique de multiples électrolytes, un plasma, un plasma exempt de plaquettes, un plasma pauvre en plaquettes ou une combinaison de ceux-ci.

5. Procédé selon la revendication 4, dans lequel la solution isotonique de multiples électrolytes est enrichie avec des excipients pharmaceutiquement acceptables.

6. Procédé selon la revendication 1, dans lequel les débris cellulaires sont éliminés de la suspension décongelée par centrifugation de la suspension décongelée à 11 270 g à 17 610 g pendant 25 à 35 minutes et isolement du surnageant.

7. Procédé selon la revendication 1, dans lequel les excipients comprennent le mannitol, le saccharose, la glycine ou des combinaisons de ceux-ci.

8. Procédé selon la revendication 1, dans lequel les plaquettes de l'étape (a) sont obtenues par thrombocytaphérèse ou par centrifugation de sang total.

9. Procédé selon la revendication 1, dans lequel les plaquettes de l'étape (a) sont obtenues par :
a. centrifugation d'au moins 10 ml de sang humain anticoagulé à 109 g à 680 g pendant 5 à 20 minutes ;
b. isolement de la couche supérieure contenant les plaquettes et centrifugation de celle-ci à 680 g à 3442 g pendant 5 à 15 minutes ; et
c. isolement de la pastille de plaquettes exempte de plasma obtenue à la fin de l'étape (b).

10. Procédé selon la revendication 9, dans lequel la centrifugation de l'étape (a) est réalisée à 382 g pendant 5 minutes et la centrifugation de l'étape (b) est réalisée à 2720 g pendant 10 minutes.

11. Dosage d'un concentré de facteurs de croissance administrable par voie intra-dermique, intra-articulaire, sous-dermique ou topique préparé par un procédé selon la revendication 1, dérivé d'environ 1250 x 10⁶ plaquettes humaines par ml, le concentré comprenant environ 900 à 2000 pg/ml de facteur de croissance épidermique (EGF), 30 à 300 pg/ml de facteur de croissance endothélial vasculaire (VEGF), de 20 à 100 pg/ml de facteur de croissance des fibroblastes basique (b-FGF), de 40 000 à 120 000 pg/ml de facteur de croissance transformant β (TGF-β) et de 200 000 à 600 000 pg/ml de facteur de croissance dérivé des plaquettes AB (PDGF-AB) suspendus dans un milieu isotonique ; d'environ 875 x 10⁶ plaquettes humaines par ml, le concentré comprenant environ 800 à 1200 pg/ml d'EGF, 20 à 80 pg/ml de VEGF, 15 à 30 pg/ml de b-FGF, 30 000 à 40 000 pg/ml de TGF-β et 100 000 à 200 000 pg/ml de PDGF-AB suspendus dans un milieu isotonique ; ou d'environ 625 x 10⁶ plaquettes humaines par ml, le concentré comprenant environ 500 à 1000 pg/ml d'EGF, 10 à 20 pg/ml de VEGF, 10 à 25 pg/ml de b-FGF, 20 000 à 30 000 pg/ml de TGF-β et 60 000 à 150 000 pg/ml de PDGF-AB suspendus dans un milieu isotonique.

12. Composition thérapeutique comprenant le concentré de facteurs de croissance administrable par voie intra-dermique, intra-articulaire, sous-dermique ou topique préparé par le procédé selon la revendication 1, pour une utilisation dans le traitement de pathologies dermatologiques, orthopédiques, neurologiques ou endocrinologiques, dans laquelle les pathologies comprennent l'alopécie androgénétique, la perte de cheveux, l'hyperpigmentation périorbitaire, les rides nasolabiales, les rides du visage, l'acné, les cicatrices liées à l'acné, les lésions chroniques, les brûlures, l'épicondylite, les ulcères du pied diabétique, les fistules et les modifications dermatologiques non voulues liées à l'âge.

13. Composition thérapeutique comprenant le concentré de facteurs de croissance administrable par voie intra-dermique, intra-articulaire, sous-dermique ou topique préparé par le procédé selon la revendication 1, dans laquelle le concentré de facteurs de croissance est combiné à des constituants supplémentaires comprenant le sang, les solutions salines, les nanoparticules d'argent, l'acide hyaluronique, les peptides immunomodulateurs, les facteurs de croissance, les hormones, les antibiotiques, les anticorps monoclonaux, les récepteurs recombinants, les véhicules ou des combinaisons de ceux-ci.
